(19) Europäisches Patentamt
European Patent Office
Office européen des brevets

(11) **EP 2 677 449 A1**

(12) **EUROPEAN PATENT APPLICATION**

(43) Date of publication:
**25.12.2013 Bulletin 2013/52**

(51) Int Cl.:
**G06F 19/22** *(2011.01)*  **G06F 19/24** *(2011.01)*

(21) Application number: **12172544.4**

(22) Date of filing: **19.06.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
• **Sjöblom, Tobias**
 **753 21 Uppsala (SE)**
• **Cheong, Ian**
 **Singapore 358416 (SG)**

(72) Inventors:
• **Sjöblom, Tobias**
 **753 21 Uppsala (SE)**
• **Cheong, Ian**
 **Singapore 358416 (SG)**

(74) Representative: **Brann AB**
 **P.O. Box 12246**
 **102 26 Stockholm (SE)**

(54) **Method and device for efficient calculation of allele ratio confidence intervals and uses thereof**

(57)  The invention relates to a method for determining the presence of a mutation in a DNA/RNA-sample comprising a plurality of DNA/RNA-fragments, the method comprising:

a) storing in a computer memory for each (f) of a number (N) of measured DNA/RNA-fragments in the sample information on:

an individual probability estimation ($p_{f,s}$) that a measured nucleotide base in a position (s) is correctly identified, and on

a match ($m_{f,s}$) between the measured nucleotide base in said position of the measured DNA/RNA-fragment and a reference nucleotide base ($r_s$) in said position (s) of a reference DNA/RNA,

b) estimating with the processor a joint probability $P_s(n)$ of measuring a specific number (n) of matching nucleotide bases at said position (s) by summing the combinations of individual probabilities ($p_{f,s}$) and reciprocal probabilities ($1-p_{f,s}$) that gives that specific number (n) of matches, and

c) determining if a mutation is present by controlling the processor to produce the total number of measured matches ($M=\Sigma_f m_{f,s}$; for s = constant) at said position with the joint probability $P_s(n)$ of measuring that number (M) of matches at said position.

The invention also relates to a computer program and to an electronic device.

Fig 1

**Description**

**TECHNICAL FIELD**

The present invention relates generally to the identification of mutations in DNA/RNA-sequences and, more particularly, to improve decision-making on whether sequence differences exist between two nucleotide sequences.

**BACKGROUND**

Detection of mutations in DNA or RNA is integral to several scientific as well as diagnostic procedures. String representations of these collected sequences, termed reads, are aligned to a reference genome using alignment software. Two principal types of errors affect bases in an aligned read: measurement errors and alignment errors, both quantified using quality scores. Several tools have been designed to determine whether a mutation exists at a position in aligned sequence data. Such tools include FreeBayes™, SAMtools™, SOAPsnp™, SnvMix™, VarScan™, SomaticSniper™, and others. Several of these tools use Bayesian statistics to calculate the probability of mutation. Bayesian methods assume that genotypes are independently and identically distributed (i.i.d.). However, the assumption of identical distribution is valid only if all factors act equally across the genome, an assertion which is problematic in the context of cancer genomes. There are many potential causes of genomic alterations violating the premise of identical distribution, such as aneuploidy, gene copy number alterations, loss of heterozygosity, fluctuations in local intrinsic mutability, and sample preparation artifacts. If these biases act non-uniformly across the genome, then only local information in close proximity to the analyzed base position would be relevant. As a result, Bayesian generalizations about the global distribution of genotypes would not be applicable to specific variations which deviate from the modeled allele frequencies. To compound the problem, tumor cell populations in clinical specimens such as those obtained from biopsies exhibit two confounding characteristics. They are clonally heterogeneous and often admixed at a low proportion to the normal cell population. What this means practically is that rare variants infrequently represented in the dataset are more likely to be mis-categorized as false negatives. Although recent progress in population-based Bayesian methods such as FreeBayes are able to take into account sequence and region-specific configuration of ploidy, these local biases must be inferred from prior information. During this process of inference, several model parameters are estimated by an iterative numerical algorithm which introduces a potential source of error. While Bayesian network models are appropriate in analyses of genomes where allele ratios are expected to be near 0.5 or 1, they are less suited in mutational analyses of tissues or diseases where the alternative allele is observed in 10% or less of the alleles in the sample. A normal state where this occurs is exemplified by the analyses of mitochondrial genomes, where each cell contains a multitude of different mitochondrial genomes. A diseased state is exemplified by the tumor tissues where allele ratios strongly deviating from 0.5 or 1 are generated by chromosomal abnormalities and ongoing clonal evolution in the tumor cells, the normal tissues contained within tumors and surrounding normal tissues present in the sample analyzed.

**SUMMARY OF THE INVENTION.**

One objective of the invention is to improve the ability to identify mutations in nucleotide sequence data.

According to one aspect of the invention this objective is achieved with the method of claim 1.

According to a second aspect of the invention this objective is achieved with a computer program according to claim 13.

The invention comprises determining the presence of a mutation in a DNA/RNA-sample comprising a plurality of DNA/RNA-fragments, the method comprising a) storing in a computer memory for each (f) of a number (N) of measured DNA/RNA-fragments in the sample information on: an individual probability estimation ($p_{f,s}$) that a measured nucleotide base in a sequence position (s) is correctly identified, and on a match ($m_{f,s}$) between the measured nucleotide base in said position of the measured DNA/RNA-fragment and a reference nucleotide base ($r_s$) in said position (s) of a reference DNA/RNA, b) estimating with the processor a joint probability $P_s(n)$ of measuring a specific number (n) of matching nucleotide bases at said position (s) by summing the combinations of individual probabilities ($p_{f,s}$) and reciprocal probabilities ($1-p_{f,s}$) that give that specific number (n) of matches, and c) determining if a mutation is present by controlling the processor to produce the total number of measured matches ($M=\Sigma_f m_{f,s}$; for s = constant) at said position and the joint probability $P_s(n)$ of measuring that number (M) of matches at said position (s).

The invention thus enables decision-making as to whether a mutation exists at a position of interest in a nucleotide sequence based on hypothesis testing involving confidence intervals of allele ratios that were derived under a minimal number of assumptions. In contrast, existing technologies compute a probability of mutation at the position of interest while introducing several assumptions that cannot be validated or invalidated. The invention further enables mutation detection in samples where a low fraction of the alleles in a sample have the mutation.

Using the individual probability estimations $p_{f,s}$ that a measured value is correct to calculate the joint probability P(n) of measuring a specific number of measured matches leads to an improved quality in the determination of a mutation due to, amongst others, that a smaller number of assumptions leading to uncertainties are needed. Yet another advantage is that the method can be used in a wider area of application, and in particular for samples with low mutant allele ratios. Preferably, the invention comprises performing the method on at least 30 or more DNA/RNA-fragments or reads. More preferably, the invention comprises performing the method on at least 50 or more DNA/RNA-fragments or reads. Most preferably, the invention comprises performing the method on at least 100 or more DNA/RNA-fragments or reads.

In one embodiment the information on the match ($m_{f,s}$) between the measured nucleotide base and the reference nucleotide base comprises information on which nucleotide base is measured in a position (s) and on the corresponding reference nucleotide base in that position (s). The method then also comprises comparing the measured nucleotide base with the reference nucleotide base. In another embodiment the information on the match ($m_{f,s}$) between the measured nucleotide base and the reference nucleotide base comprises information on whether the nucleotide bases of the measured nucleotide base and the reference nucleotide base are of the same or different types. A match is then ascribed a positive value, or yes-value, when the nucleotide bases have the same type. Preferably, the step of estimation of the joint probability $P_s(n)$ by summing the combinations of individual probabilities ($p_{f,s}$) and reciprocal probabilities ($1-p_{f,s}$) comprises using a reciprocal probability in case there is no match between the measured nucleotide base and the reference nucleotide base, that is, when the measured nucleotide base and the reference nucleotide base in the given position are of different types. In one embodiment the invention comprises discarding any measurements of non-matching values occurring with a lower frequency than the highest frequency of non-matching values. The joint probability is thus an estimation of the probability to measure a certain number of positive matches from the entire sample of DNA/RNA-fragments. The term position is used to mean a position in a nucleotide base sequence. Normally the position refers to a nucleotide base in the nucleotide base sequence of the reference DNA/RNA.

In one embodiment, the individual probability estimate comprises receiving a quality score from alignment software. The invention then comprises converting the quality score to an estimated probability that the received match is correctly measured. In one embodiment, the individual probability estimate $p_f$ is estimated as $p=1-10^{(-x/10)}$ where x is the quality score.

In a preferred embodiment, in the summing of combinations of individual and reciprocal probabilities step, the term combination refers to the standard mathematical definition of a combination (according to combinatorics, that is C (n,k)).

In one embodiment, confidence intervals for allele ratios in datasets comprising multiple observations of a given base position in a DNA sample, where each base observation is associated with a probability of correct base call and alignment, are first computed. The presence of a mutation can then be determined by hypothesis testing. In a second step, allele ratio confidence intervals from two different samples are used in a decision-making process to determine whether differences exist between the two samples.

According to another embodiment, the invention comprises controlling the processor to repeat step b) to estimate at least one additional joint probability $P_s(n)$ of measuring another, specific number (n) of matching nucleotide bases, and controlling the processor to sum one or more joint probabilities $P_s(n)$ with specific numbers (n) of matching nucleotide bases about the measured number of matches (M) to estimate a confidence that a mutation is present.

According to yet another embodiment, the invention comprises allocating a data structure Z in the digital memory having a plurality of data entries ($Z_x$), and storing in each data entry ($Z_x$) the combinations of products of individual probability estimations ($p_{f,s}$) comprising the same number (x) of product terms. In another embodiment, each data entry $Z_x$ may instead comprise a row of data variables ($z_{x,s}$), wherein each variable comprises the value of one combination of individual probability estimations.

[Paragraph originally here moved further below. Reason: speed advantages pertain to data structure A, not Z.]

According to yet another embodiment, the invention comprises storing a combination distribution weight function ($B_{x,n}$) in the computer memory, and controlling the processor to estimate the joint probability $P_s(n)$ as a weighted sum of values of the data entries ($Z_x$) of Z multiplied with the combination distribution weight function ($B_{x,n}$). According to yet another embodiment, the invention comprises storing a combination distribution weight function ($B_{x,n}$) comprising a series of binomial coefficients with alternating signs in the digital memory. This enables an exact calculation of the distribution weight function.

In another embodiment, the invention comprises allocating a data structure A in the digital memory having a plurality of data entries ($a_{x,n}$), storing in the data entries ($a_{x,n}$) of the data structure A the value of one combination of terms of the individual probability estimates ($p_{f,s}$), and calculating the data entries ($Z_x$) of Z as a sum of values of data entries ($a_{x,n}$) of the data structure A.

By allocating and storing a data structure A containing the combinations of products of the terms of the individual probability estimations it is possible to decrease the number of computations needed to estimate the joint probabilities $P_s(n)$ of measuring a specific number of measured matches. Specifically, the dynamic programming character of the data structure A groups computations efficiently to eliminate redundancy. Thus, the total number of computations to populate data structure A increases in proportion to $N^2$ and is described by the arithmetic sum $(N/2)(1+N)$ whereas exhaustively enumerating all combinatorial probability estimations $(p_{f,s})$ would impose an exponentially increasing computational burden, scaling in proportion to $2^{N+1}$. As an example, when $N = 1000$, utilizing data structure A would require approximately 500,500 computations in comparison to approximately $10^{300}$ using exhaustive enumeration. For the foregoing reasons, efficiency and computational economy is improved.

Through reuse of previously calculated values, the speed of initialization and calculation of entries in the data structure A can be dramatically increased while less computer memory is needed.

In yet another preferred embodiment, the invention comprises assigning values to some of the data entries $(a_{x,n})$ of the data structure A by initializing a first row of data entries $(a_{1,n})$ of the data structure A by assigning to each data entry $(a_{1,n})$ the value of one individual probability estimate $(p_f)$.

In another embodiment, the invention comprises assigning values to some of the data entries $(a_{x,n})$ of the data structure A, and controlling the processor to calculate and assign values to the remaining data entries $(a_{x,n})$ of the data structure A by using values of previously initialized data entries $(a_{x,n})$ of the data structure A. Preferably, the invention also comprises: controlling the processor to calculate and assign values to the remaining data entries $(a_{x,n})$ of the data structure A by using values of previously initialized data entries $(z_x)$ of the data structure Z.

In another embodiment, the invention comprises controlling the processor to calculate and assign values to the remaining data entries $(a_{x,n})$ of the data structure A recursively. This entails that the assigning of values of combinations to each data entry of A comprises assigning combinations both forwardly and recursively in parallel. Preferably, the invention also comprises controlling the processor to calculate and assign values to the remaining data entries $(a_{x,n})$ of the data structure A by using values of previously initialized data entries $(z_x)$ of the data structure Z.

In yet another embodiment, the invention comprises that the individual probability estimations $(p_{f,s})$ that a measured nucleotide base is correct is determined based on an alignment quality for each DNA/RNA fragment and on a measurement error estimation for each individual nucleotide base position of the DNA/RNA-fragment.

In yet another embodiment, the invention comprises a computer program comprising a sequence of computer program instructions adapted to control a computer comprising a processor and at least one computer memory associated with the processor to determine a presence of a mutation in a DNA/RNA-sample comprising a plurality of DNA/RNA-fragments, the computer program controlling the computer to perform, for one or more position(s) of the nucleotide sequences of said DNA/RNA-fragments in the sample, the steps: (a) storing in the computer memory for each (f) of a number (N) of measured DNA/RNA-fragments in the sample information on: a probability estimation $(p_{f,s})$ that a measured nucleotide base in a position (s) is correctly identified, and on a match $(m_{f,s})$ between the measured nucleotide base in said position of the measured DNA/RNA-fragment and a reference nucleotide base $(r_s)$ in said position (j) of a reference DNA/RNA, (b) estimating with the processor a joint probability $P_s(n)$ of measuring a specific number (n) of matching nucleotide bases at said position (j) by summing the combinations of individual probabilities $(p_{f,s})$ and reciprocal probabilities $(1-p_{f,s})$ that give that specific number (n) of matches, and (c) determining if a mutation is present by controlling the processor to produce the total number of measured matches $(M=\Sigma m_{f,s};$ for s = constant) at said position with the joint probability $P_s(n)$ of measuring that number (M) of matches at said position.

In yet another embodiment, the invention comprises a computer program that is adapted to control the computer to perform the steps: controlling the processor to repeat step b) to estimate at least one additional joint probability $P_s(n)$ of measuring another, specific number (n) of matching nucleotide bases, and controlling the processor to sum one or more joint probabilities $P_s(n)$ with specific numbers (n) of matching nucleotide bases about the measured number of matches (M) to estimate a confidence that a mutation is present.

In yet another embodiment, the invention comprises a computer program that is adapted to control the computer to perform the steps: allocating a data structure Z in the digital memory having a plurality of data entries $(Z_x)$, and storing in each data entry $(Z_x)$ the combinations of products of individual probability estimations $(p_{f,s})$ comprising the same number (x) of product terms.

In yet another embodiment, the invention comprises a computer program that is adapted to control the computer to perform the steps: storing a combination distribution weight function $(B_{x,n})$ in the computer memory, and controlling the processor to estimate the joint probability $P_s(n)$ as a weighted sum of values of the data entries $(Z_x)$ of Z multiplied with the combination distribution weight function $(B_{x,n})$.

In yet another embodiment, the invention comprises a computer program that is adapted to control the computer to perform the step: storing a combination distribution weight function $(B_{x,n})$ comprising a series of binomial coefficients with alternating signs in the digital memory.

In yet another embodiment, the invention comprises a computer program that is adapted to control the computer to perform the steps: allocating a data structure A in the digital memory having a plurality of data entries $(a_{x,n})$, storing in the data entries $(a_{x,n})$ of the data structure A the value of one combination of terms of the individual probability estimates $(p_{f,s})$, and calculating the data entries $(Z_x)$ of Z as a sum of values of data entries $(a_{x,n})$ of the data structure A.

In yet another embodiment, the invention comprises a computer program that is adapted to control the computer to perform the step: assigning values to some of the data entries ($a_{x,n}$) of the data structure A by initializing a first row of data entries ($a_{1,n}$) of the data structure A by assigning to each data entry ($a_{1,n}$) the value of one individual probability estimate ($p_f$).

In yet another embodiment, the invention comprises a computer program that is adapted to control the computer to perform the steps: assigning values to some of the data entries ($a_{x,n}$) of the data structure A, and controlling the processor to calculate and assign values to the remaining data entries ($a_{x,n}$) of the data structure A by using values of previously initialized data entries ($a_{x,n}$) of the data structure A.

In yet another embodiment, the invention comprises a computer program that is adapted to control the computer to perform the step: controlling the processor to calculate and assign values to the remaining data entries ($a_{x,n}$) of the data structure A recursively.

In yet another embodiment, the invention comprises a computer program characterized in that the information on the individual probability estimations ($p_{f,s}$) that a measured nucleotide base is correct is determined based on an alignment quality for each DNA/RNA fragment and on a measurement error estimation for each individual nucleotide base position of the DNA/RNA-fragment.

In yet another embodiment, the invention comprises performing the steps a) to c) for a second DNA/RNA-sample comprising DNA/RNA-fragments containing sequences with substantially the same nucleotide base positions as the first sample, and d) determining if a mutation is present by comparing the results obtained in c) for the first and second samples.

In yet another embodiment, the invention comprises that the first and second DNA/RNA-samples originates from healthy and tumor tissue from the same individual, respectively, and that the method comprises determining a cancerous state if the nucleotide bases in at least one corresponding position (s) differs between the first and second DNA/RNA-samples within a desired level of confidence.

In a third aspect of the invention, it comprises an electronic device comprising analog and/or digital electronic circuitry for determining the presence of a mutation in a DNA/RNA-sample comprising a plurality of DNA/RNA-fragments, the device comprising: a first storage module adapted to store, for each (f) of a number (N) of measured DNA/RNA-fragments in the sample, information representing an individual probability estimation ($p_{f,s}$) that a measured nucleotide base in a position (s) is correctly identified, and information representing a match ($m_{f,s}$) between the measured nucleotide base in said position of the measured DNA/RNA-fragment and a reference nucleotide base ($r_s$) in said position (s) of a reference DNA/RNA; an estimation module adapted to estimate a joint probability $P_s(n)$ of measuring a specific number (n) of matching nucleotide bases at said position (s) by summing the combinations of individual probabilities ($p_{i,j}$) and reciprocal probabilities ($1-p_{f,s}$) that gives that specific number (n) of matches, and a determination module adapted to determine if a mutation is present by producing the total number of measured matches ($M=\Sigma m_{f,s}$; for s = constant) at said position with the joint probability $P_s(n)$ of measuring that number (M) of matches at said position.

BRIEF DESCRIPTION OF THE DRAWINGS.

Fig 1 is a block diagram of the method for computation of combinatorial probability mass functions and their confidence intervals of the invention.

Figs 2a, 2b, and 2c are diagrams of software implementations for computing $Z_x$, the combinations of products of individual probability estimations ($p_{f,s}$) comprising the same number (x) of product terms of the invention.

Fig 3 is a diagram of a software implementation for computing the probabilities P(0) to P(N) from binomial coefficients and $Z_x$ and an example for implementing the combination distribution weight function ($B_{x,n}$) given 6 input values for individual probability estimation ($p_{f,s}$).

Fig 4 is a diagram of actual performance of conventional enumeration versus use of the method of the invention to compute combinatorial probability mass functions.

Fig 5 is four diagrams of probability mass functions of reference allele ratios at four different base positions in different breast cancer samples.

Fig 6 is a comparison of the mutation detection sensitivity of the confidence interval based method of the invention to a conventional Bayesian method (FreeBayes) at 90 % and 95 % observed reference allele ratios, respectively.

Fig 7 is a block diagram of an approach to identify mutations present in a first sample but not in a second sample or vice versa using confidence intervals of allele ratios.

Fig 8 is two diagrams of probability mass functions of reference allele ratios at the same base position in DNA samples derived from tumor and normal tissue, respectively, of the same individual.

## DETAILED DESCRIPTION OF THE INVENTION.

In a workflow of DNA sequence analysis, a sample is subjected to sequence analyses in an instrument that produces multiple independent observations (sequence reads) of the bases of interest. Alignment software is used to match the independent sequence reads to a reference genome, while producing a quality score reflecting alignment quality as well as base quality. Two principal types of errors affect bases in an aligned read: measurement errors and alignment errors, both quantified using quality scores. While each base in a read is associated with its own measurement error, all bases of a read will share the same alignment quality score. In such sequence datasets, the probability that any observation correctly identifies the base is implied by the corresponding alignment quality score. For example, the quality scores of the Mosaik alignment software is designed so that base calls or positions given a quality score of x should be correct on average with relative frequency $1-10^{(-x/10)}$, which is an estimated probability. Further, the combinatorial probability of observing the reference base n number of times can be computed as P(n) the summed probabilities of all possible combinations that yield n observations. The time complexity of conventional combinatorial probability computations is exponential, which limits their practical applicability to n < 30 observations, while several thousands of observations of each base may be collected in the course of DNA sequencing.

In one aspect of the invention, the number of observed bases corresponding to the expected reference base and the otherwise most frequently observed base at each position of interest are considered; observations of other bases are by convention assumed to be erroneous and therefore discarded. The fraction of reference base observations of total observations is defined as the allele ratio. The allele ratio indicates whether the observed base in a position differs from the reference base, and such a difference can be interpreted as a mutation. First, a confidence interval is created for the allele ratio from the number of observations at a given position that are identical to the reference base and those identical to the otherwise most frequently observed base. Second, the probabilities to observe different numbers of the reference base are calculated from the alignment quality scores, which are estimated probabilities. Third, the probability to observe any given number of the reference base is computed by summing the probabilities of all possible combinations yielding the given number. The probability of a combination of bases at the position is equal to the product of the probabilities for each base, which is estimated by the alignment quality score. As other bases than the reference base and the otherwise most frequently observed base have been discarded, the probability of the base deviating from the reference base is one minus the probability estimated by the alignment score when the observed base is different from the reference base. The method used to calculate and sum all probabilities is described in the following section. Finally, the confidence interval is widened until the probability to observe a value in the interval is greater than or equal to the desired confidence level. By dividing the endpoint values of the confidence interval with the total number of observations, a confidence interval for the allele ratio is obtained.

Suppose that the nucleotide of interest has been sequenced at a depth of four reads, that the reference allele is A, and that the observed alleles are as follows:

| Read number | 1 | 2 | 3 | 4 |
|---|---|---|---|---|
| *Observed allele* | A | A | A | T |
| *Quality score* | 10 | 30 | 7 | 10 |
| *Implied probability of reference allele* | 0.9 | 0.999 | 0.8 | 0.1 |

The observed allele ratio is 0.75, i.e. three observations of the reference allele to one observation of the alternative allele. Set the probabilities to observe the reference allele in the four different reads to $p_1 = 0.9$, $p_2 = 0.999$, $p_3 = 0.8$ and $p_4 = 0.1$. The probabilities to observe X number of reference alleles (P(X)) can then be calculated using the following probabilistic combinatorial calculation:

$$P(0) = (1-p_1)(1-p_2)(1-p_3)(1-p_4) = 0.000018$$

$$P(1) = p_1(1-p_2)(1-p_3)(1-p_4) + p_2(1-p_1)(1-p_3)(1-p_4) + p_3(1-p_1)(1-p_2)(1-p_4) + p_4(1-p_1)(1-p_2)(1-p_3) = 0.018218$$

$$P(2) = p_1p_2(1-p_3)(1-p_4) + p_1p_3(1-p_2)(1-p_4) + p_1p_4(1-p_2)(1-p_3) + p_2p_3(1-p_1)(1-p_4) + p_2p_4(1-p_1)(1-p_3)$$

$$+ p_3p_4 (1-p_1)(1-p_2) = 0.236438$$

$$P(3) = p_1p_2p_3(1-p_4) + p_1p_2p_4(1-p_3) + p_1p_3p_4(1-p_2) + p_2p_3p_4(1-p_1) = 0.673398$$

$$P(4) = p_1p_2p_3p_4 = 0.071928$$

This will result in a distribution of implied probabilities of observing allele ratios as follows:

| Allele ratio | 0 | 0.25 | 0.5 | 0.75 | 1 |
|---|---|---|---|---|---|
| Probability | 0.00002 | 0.0182 | 0.2364 | 0.6734 | 0.0719 |

Now, consider the two confidence intervals for the observed allele ratio [0.5; 1] and [0.25; 1]. The two confidence intervals have confidence levels

$$P(a/n \in [0.5; 1]) \approx 0.9818 > 0.95, \text{ i.e. } 95\,\%,$$

$$P(a/n \in [0.25; 1]) \approx 0.99998 > 0.999, \text{ i.e. } 99.9\,\%.$$

Thus, the hypothesis that the true allele ratio of the nucleotide is zero can be rejected at the 99.9 % level, while the hypothesis that the true allele ratio is 0.25 can be rejected at the 95 % level.

The allele ratio can indicate whether an observed base at the position is different from the reference base, which could be interpreted as a potential mutation. The method of the present invention is applied to aligned sequences and their associated quality scores to efficiently construct a confidence interval for the allele ratio at each base position at a desired confidence level. With the invention a more efficient way of generating combinatorial probability distributions than by exhaustive enumeration has been achieved.

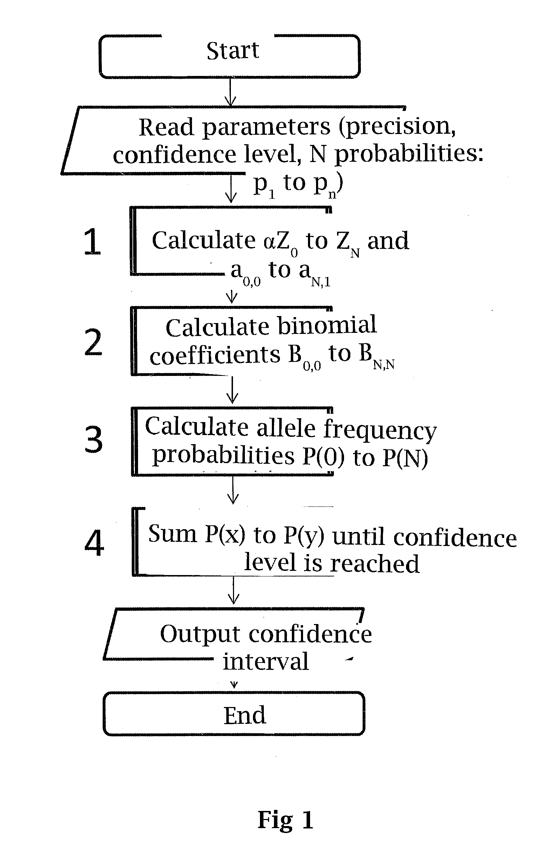

Figure 1 is a flow chart of the part of the method of the invention that computes confidence intervals of allele ratios. This part of the method can be divided in six steps.

In a first step, the desired confidence level and precision along with the independent probabilities $p_1$ to $p_N$ are provided as inputs to the method.

Given N total probabilities for any specific base, the probability P(n) is expressed as a product series of binomial coefficients and a variable $Z_x$ defined as the sum of all possible x-tuple multiplicants from among the N probabilities.

$$P(n) = \binom{n}{0}.Z_n - \binom{n+1}{1}.Z_{n+1} + \binom{n+2}{2}.Z_{n+2} - \binom{n+3}{3}.Z_{n+3} \ldots \binom{N}{N-n}.Z_N$$

In a second step, an N x N upper triangular matrix Z composed of the sums $Z_x$ representing all unique combinations of x drawn from $p_1, p_2 \ldots p_N$ is generated.

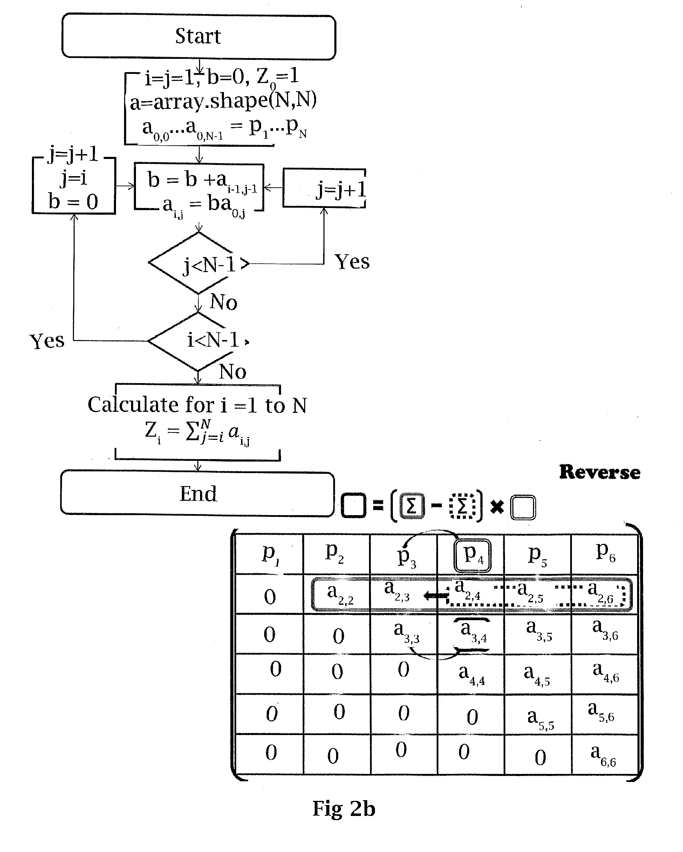

In a preferred embodiment of the invention, a recursion is first used to calculate the values of Z. For every integer from 1 to the number of probabilities to be computed, a-values are first calculated by using a-values from the previous recursion and the probabilities supplied to the method. The Z-values are then computed using a-values and the supplied probabilities (Figure 2B). Details of this calculation are hereby given.

Calculation of the Z-values is based on the principle that all possible combinations drawn from the supplied probabilities may be represented by a binomial tree and that the self-similarity inherent in this tree enables a dynamic programming approach which avoids redundant calculations. Conceptually, any binomial tree with depth $x \geq 2$ may be described as two identical subtrees with one ($^{left}B_{x-1}$) connected to the root and the other ($^{right}B_{x-1}$) connected to a progeny $p_x$ of that root and thus offset by one depth level. Using this principle, construct a binomial tree $B_N$ incrementally from x=1 to N with root value 1. $B_N$ represents all possible ways of choosing 1, 2, 3...N out of a total of N independent probabilities. Each node in $B_N$ is labeled with an independent probability ($p_1, p_2, \ldots p_f \ldots p_N$ ) and the value of each node is the product of its labeled probability and all its ancestral probabilities. Each node thus represents the value of a unique multiplicative combination among $p_1$ to $p_N$. If $S(B, k) = \Sigma$(nodal values at depth k within binomial tree B), we state the following property (the "similar subtree property") for any two identical subtrees within $B_N$.

$$S(^{left}B_{x-1}, k) = p_x . S(^{right}B_{x-1}, k)$$

From the above, it follows that (a) every node in $B_N$ at depth > 2 belongs exclusively to one unique $S(^{right}B_{x-1}, k)$ block and (b) the set of nodes in $B_N$ and the set of nodes in all $S(^{right}B_{x-1}, k)$ blocks within $B_N$ are equivalent. Because all N nodal values for depth x = 1 within $B_N$ are defined (i.e. $p_1...p_N$), the values of all $S(^{right}B_{x-1}, k)$ may be derived recursively using the above relation. Since the sum of all nodal values for depth x within $B_N$ will give the result for $Z_x$, the summation of all $S(^{right}B_{x-1}, k)$ blocks at depth x within $B_N$ will return the same. There are many possible variations to use the similar subtree property for calculation of combinatorial probability distributions and we present three main embodiments. Computer pseudocode for implementing this approach is presented in Example 1.

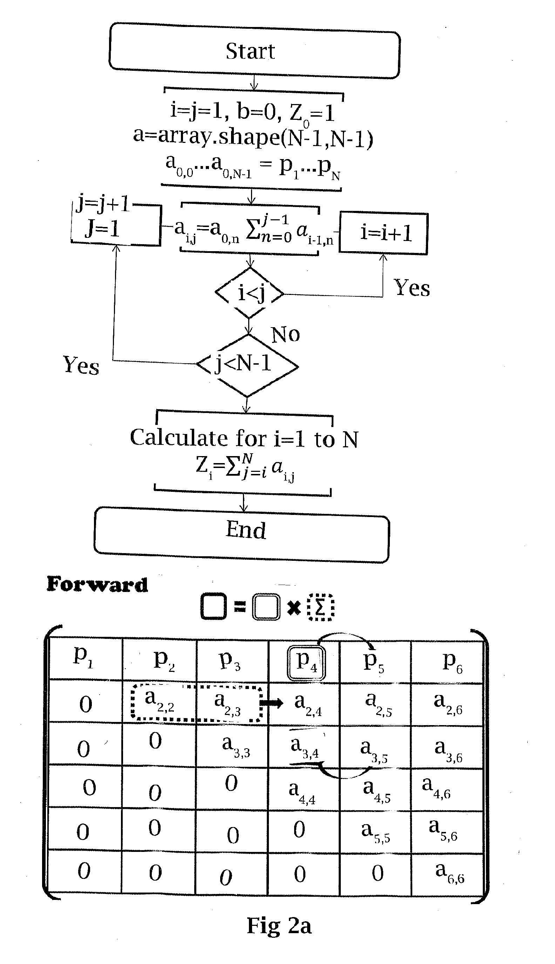

In one embodiment (hereinafter referred to as "Forward calculation") an N by N upper triangular matrix A is first initialized and the first row populated with $p_1$ to $p_N$. The rest of the matrix elements ($a_{2,2}...a_{N,N}$) are then generated by software described structurally by the flowchart in Fig 2A. Very briefly, to calculate any element $a_{i,j}$, sum all elements one row above and to the left ($a_{i-1,i-1}$ to $a_{i-1,j-1}$) and multiply this sum by the corresponding $p_1...p_N$ directly above ($a_{o,j}$). An example for N=5 is depicted diagrammatically in Fig 2A. In this example, calculation of elements $a_{2,2}...a_{N,N}$ elements are generated directionally from top to bottom rows and within each row, from left to right. Arrows depict the translation (curved arrow) or expansion (straight arrow) of the relative windows for calculation of the next element. Computer pseudocode for implementing this approach is presented in Example 2.

In another embodiment (hereinafter referred to as "Reverse calculation") an N by N upper triangular matrix is first initialized and the first row populated with $p_1$ to $p_N$. The rest of the matrix elements ($a_{2,2}...a_{N,N}$) are then generated by software described structurally by the flowchart in Fig 2B. Very briefly, to calculate any element $a_{i,j}$, take the difference of the sum all elements one row above ($a_{i-1,i-1}$ to $a_{i-1,N}$ a.k.a. $Z_{i-1}$) and the sum of all elements spanning from $a_{i-1,N}$ to $a_{i-1,j-1}$. Multiply this difference by the corresponding $p_1...p_N$ directly above ($a_{o,j}$). An example for N=5 is depicted diagrammatically in Fig 2B. In this example, calculation of elements $a_{2,2}...a_{N,N}$ elements are generated directionally from top to bottom rows and within each row, from right to left. Arrows depict the translation (curved arrow) or expansion (straight arrow) of the relative windows for calculation of the next element.

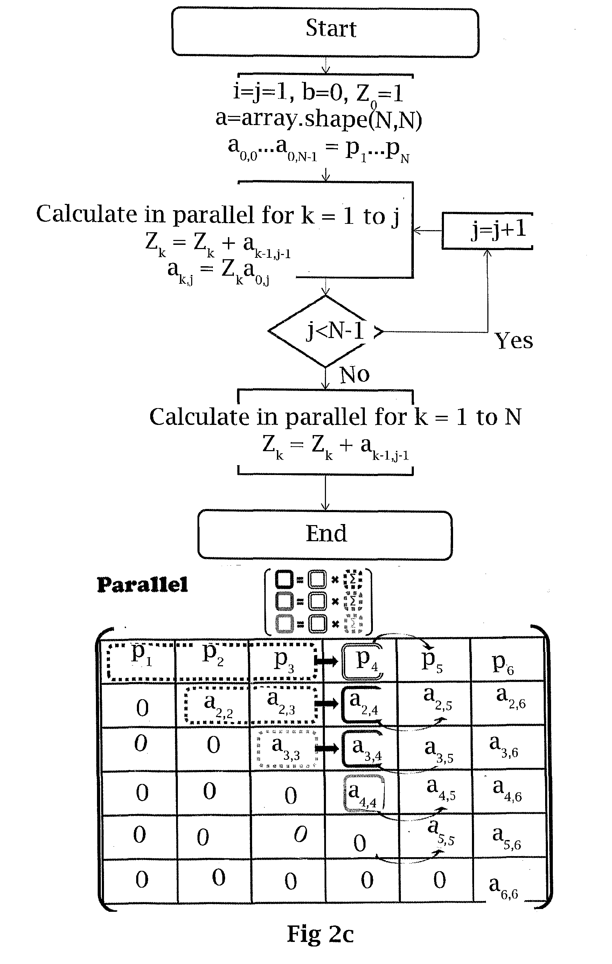

In the third embodiment (hereinafter referred to as "Parallel calculation") an N by N upper triangular matrix is first initialized and the first row populated with $p_1$ to $p_N$. The rest of the matrix elements ($a_{2,2}...a_{N,N}$) are then generated by software described structurally by the flowchart in Fig 2C. This approach is a parallelized version of the forward approach. Very briefly, we use the forward approach to calculate all columns in parallel, starting with $a_{.,1}$ and ending with $a_{.,N}$. An example for N=5 is depicted diagrammatically in Fig 2C. Arrows depict the translation (curved arrow) or expansion (straight arrow) of the relative windows for calculation of the next element.

In a third step, a matrix B is initialized with binomial coefficients which may carry either a positive or negative sign. This initialization may be computed or obtained from a table stored in memory or on disk. Several embodiments are advantageous depending on the hardware implementation of the method and the size of N.

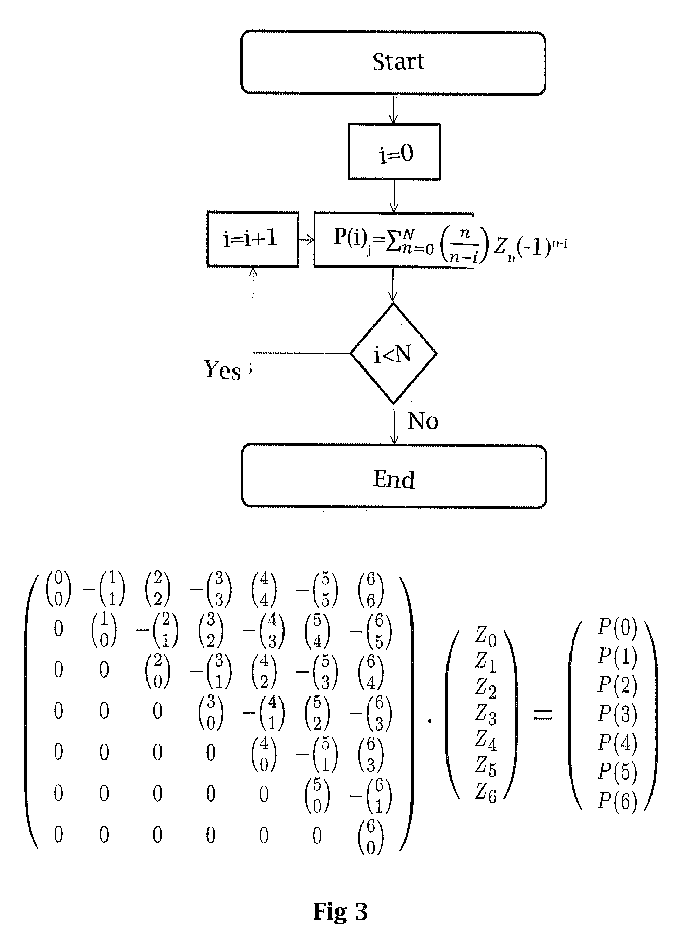

In one embodiment, B or parts of B are generated using combinatorial functions. In this embodiment, values for C(n, k) are pre-computed using a convenient formula (e.g. Pascal's rule or the Gamma function) and stored in memory or on disk, allowing quick retrieval of the relevant binomial coefficients from B. In another embodiment, B is pre-computed and structured as a two-dimensional upper triangular matrix B of N x N binomial coefficients of alternating signs. This allows the calculation of $P_s(n)$ as the dot product of B and data structure Z (Fig 3). In another embodiment, vectors representing consecutive rows of elements of the matrix *B* are sequentially generated by adding elements in the present and immediately preceding row of *B,* used to compute a product with *Z* and to generate the next row of B, and thereafter deleted from computer memory to minimize the computational effort to generate the elements of *B* as well as the memory occupancy of *B.*

In a fourth step, the probabilities $P_0$ to $P_N$ are obtained by multiplying the arrays Z and *B.*

In a fifth step, a confidence interval is created using a middle-out approach. As starting point, the frequency most likely to have the highest probability in the confidence interval is computed. Next, the method calculates probabilities towards each end of the confidence interval until the desired confidence interval limit is reached. Alternatively, the confidence interval is symmetrically widened until the probability to observe a value in the interval is greater than or equal to the desired confidence level.

In a sixth step, the upper and lower bounds of said confidence interval constitute the output of the method.

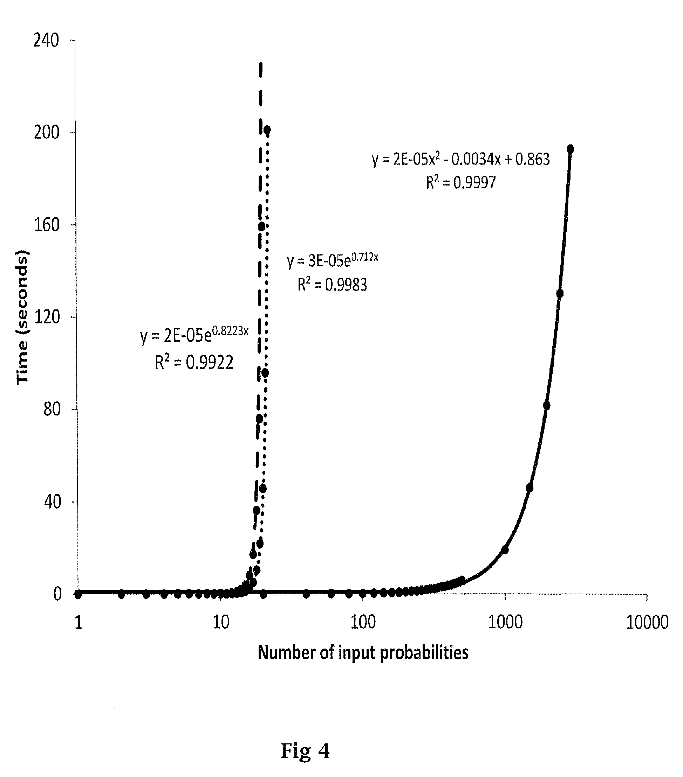

A comparison between conventional exhaustive enumeration and the method of the invention is presented in Example 3 and Fig 4.

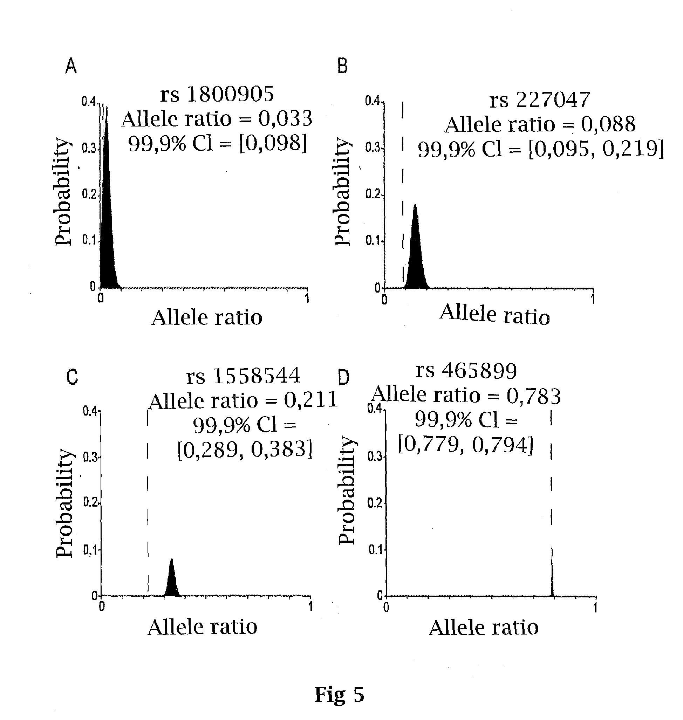

The method of the invention enables computation of probability distributions from sequence datasets with high coverage (Example 4 and Fig 5). For all cases in Example 4, no conventional enumeration-based calculations of probability distributions could be completed in practical time using a personal computer.

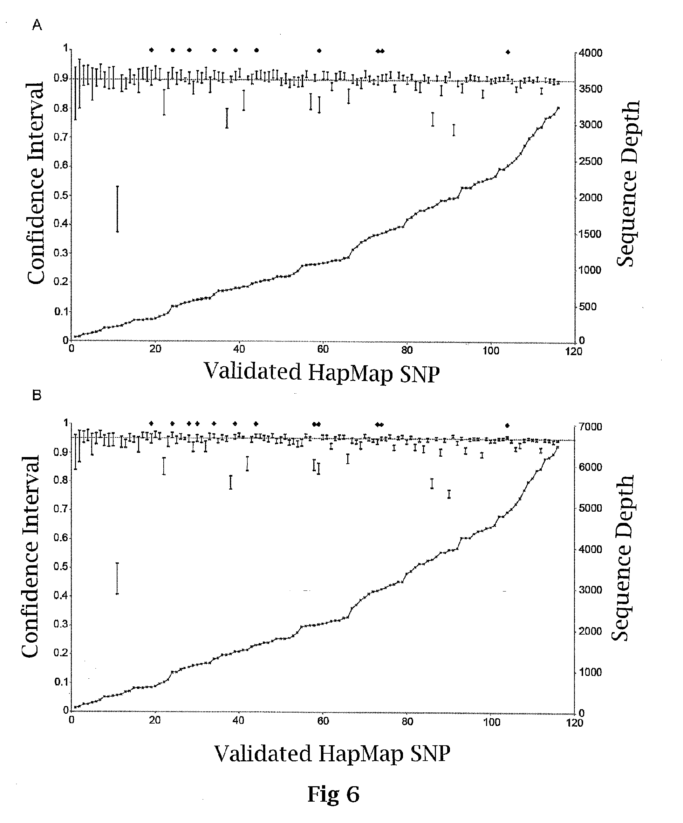

To compare the sensitivity of one embodiment of the invention to a Bayesian alternative method (FreeBayes) at low mutant allele ratios, we iteratively sampled sequence reads from breast cancer specimens in Example 4 at positions with known constitutional polymorphisms to generate 90% (A) or 95% (B) observed reference allele ratio (Fig 6). The positions were ordered by sequence depth and the 99.9% confidence interval plotted. Diamonds represent mutations identified by FreeBayes; crosses represent the number of sequences in the sample.

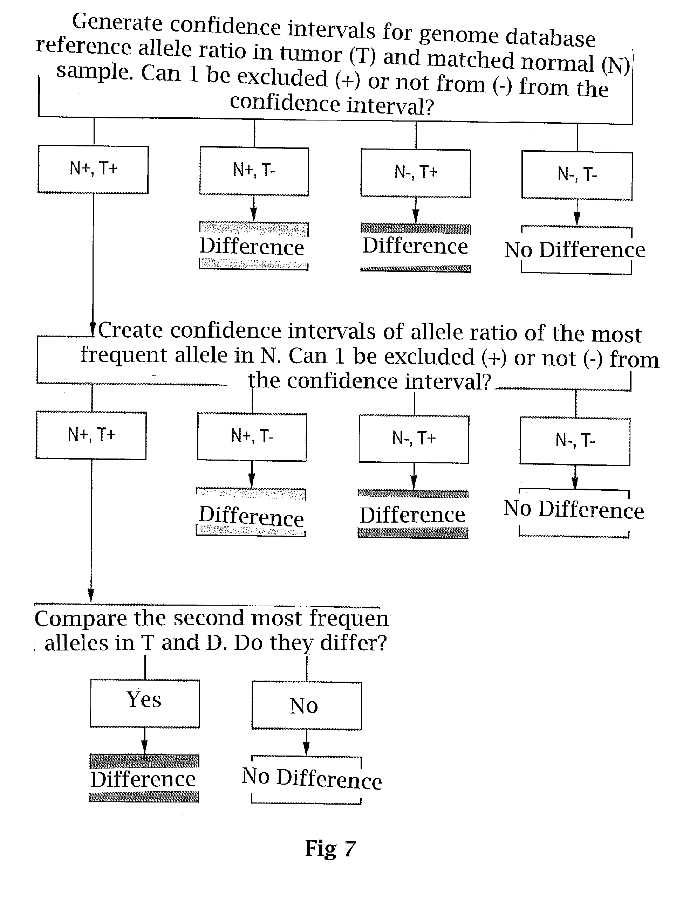

The confidence intervals obtained using embodiments of the invention can be used in a decision-making process to decide whether a mutation exists at a nucleotide position in one sample but not in the other (Example 5, Fig 7). One important application of such processes is in identification of tumor-specific (somatic) mutations by using allele ratio confidence intervals obtained at the same base position from normal and tumor DNA of the same individual. When analyzing a large number of base positions in this fashion, it is convenient to rank order the positions for example by one of the confidence limits or the confidence interval midpoint of the reference allele ratio in the tumor DNA sample. The rank order then provides a validation order for applying additional analyses. Further, the relation of confidence interval limits to a user-defined cut-off value can be used to filter false positive somatic mutations at the expense of not being able to identify mutations at base positions having reference allele ratio confidence interval including or higher than said cut-off value.

Statistical hypothesis testing at a desired confidence level (ie. 99.9%) can be used to test whether differences exist between two DNA samples at a base position. Here, this is exemplified in fig. 7 by the comparison of tumor DNA and normal DNA at a given genomic position where the two samples were obtained from the same individual with the purpose of identifying mutations present only in the one but not in the other (Fig 7). To test whether an allele ratio is equal to 1, a confidence interval for the allele ratio is created and if the confidence interval includes 1 the hypothesis is accepted (negative test) but if 1 is not in the interval the hypothesis is rejected (positive test). The reference allele is initially derived from a reference genome database. At the given position in aligned sequence data, tests are performed to determine if the tumor and patient-matched normal and tumor sample, respectively, have allele ratios of 1. This has three possible outcomes: (1) both tests are negative, meaning that both samples are homozygous for the reference allele; (2) one test is positive and the other is negative, implying a difference between tumor and normal sample at the position; and (3) both tests are positive, meaning that it is not possible to decide whether there is a difference at the position without further testing. The third case may, for instance, arise when somatic mutations occur at a position where the normal sample is heterozygous for a single nucleotide variant. In a second round of tests, the reference allele is then exchanged for the other most frequently observed allele in the normal sample and confidence intervals for the allele ratios of this most frequent allele are computed. Heterozygosity or homozygosity for this allele is determined by testing whether 0.5 or 1, respectively, is within the confidence interval of the allele ratio. If the normal sample is homozygous, test whether the allele ratio of the tumor biopsy is 1. If this test is negative, there is no evidence for differences between tumor and normal at the position; if positive, there is evidence for a difference. If the normal sample is heterozygous, test whether 0.5 is within the allele ratio confidence interval of the tumor sample. If the test is (a) negative and the tumor and normal samples have different B-alleles, there is evidence for a difference at the position; (b) negative but the tumor and normal samples have the same B-alleles, there is no evidence for a difference at the position; and (c) positive there is evidence for a difference at the position.

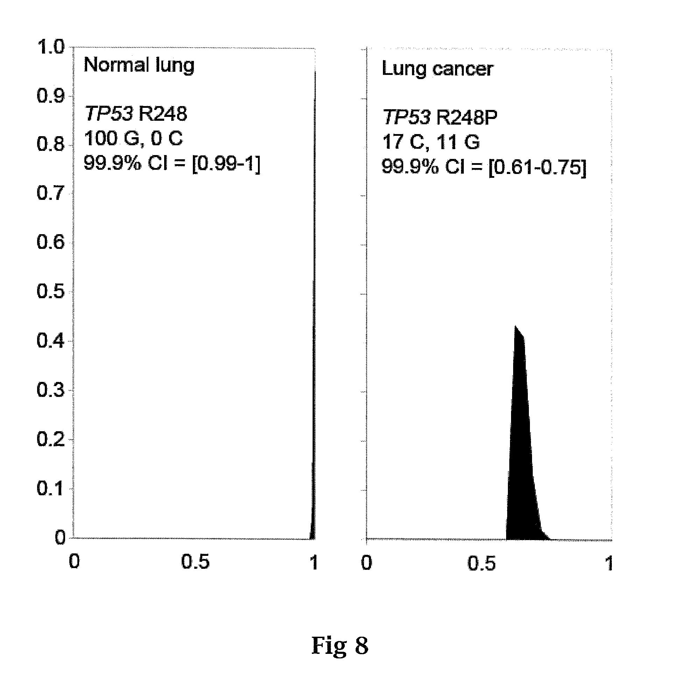

To validate this decision-making process, we analyzed sequence data from twenty-seven cancer genes in a patient-matched tumor-normal pair from a lung cancer patient using the invention (Fig 8). When mutations exclusive to the tumor sample were ranked by allele ratio, an R248P hotspot mutation in TP53 known from lung cancer was identified among the top mutations.

EXAMPLES

### Example 1.

Given N total probabilities for any specific base, the probability $P(n)$ is expressed as a product series of binomial coefficients and a variable $Z_x$ defined as the sum of all possible x-tuple multiplicants from among the N probabilities.

$$P(n) = \binom{n}{0}.Z_n - \binom{n+1}{1}.Z_{n+1} + \binom{n+2}{2}.Z_{n+2} - \binom{n+3}{3}.Z_{n+3} \ldots \binom{N}{N-n}.Z_N$$

Next, we rapidly compute the values of $Z_n$ to $Z_N$ using a self-populating matrix.

Step 1: Construct a $N \times N$ upper triangular matrix and populate the first row.

$$a_{1,*} = [p_1, p_2, p_3 \ldots p_N]$$
$$Z_1 = \sum_{j=1}^{N} a_{1,j}$$

Step 2: Recursively compute the other elements.

$$
\begin{aligned}
&\text{For } i = 2 \text{ to } N \\
&\qquad k = 0 \\
&\qquad \text{For } j = i \text{ to } N \\
&\qquad\qquad k = k + a_{i-1,j-1} \\
&\qquad\qquad a_{i,j} = k a_{1,j} \\
&\qquad \text{Next } j \\
&\qquad Z_i = \sum_{j=i}^{N} a_{i,j} \\
&\text{Next } i
\end{aligned}
$$

The matrix for $N = 6$ is given below.

$$
\begin{pmatrix}
p_1 & p_2 & p_3 & p_4 & p_5 & p_6 \\
0 & p_2 \sum_{j=1}^{1} a_{1,j} & p_3 \sum_{j=1}^{2} a_{1,j} & p_3 \sum_{j=1}^{3} a_{1,j} & p_4 \sum_{j=1}^{4} a_{1,j} & p_5 \sum_{j=1}^{5} a_{1,j} \\
0 & 0 & p_3 \sum_{j=2}^{2} a_{2,j} & p_4 \sum_{j=2}^{3} a_{2,j} & p_5 \sum_{j=2}^{4} a_{2,j} & p_6 \sum_{j=2}^{5} a_{2,j} \\
0 & 0 & 0 & p_4 \sum_{j=3}^{3} a_{3,j} & p_5 \sum_{j=3}^{4} a_{3,j} & p_6 \sum_{j=3}^{5} a_{3,j} \\
0 & 0 & 0 & 0 & p_5 \sum_{j=4}^{4} a_{4,j} & p_6 \sum_{j=4}^{5} a_{4,j} \\
0 & 0 & 0 & 0 & 0 & p_6 \sum_{j=5}^{5} a_{5,j}
\end{pmatrix}
\cdot
\begin{pmatrix} 1 \\ 1 \\ 1 \\ 1 \\ 1 \\ 1 \end{pmatrix}
=
\begin{pmatrix} Z_1 \\ Z_2 \\ Z_3 \\ Z_4 \\ Z_5 \\ Z_6 \end{pmatrix}
$$

**Example 2.**

Alternative methods based on the same principle as Example 1 can be constructed and one such alternative example is given below.

Step 1: Construct a $N \times N$ upper triangular matrix and populate the first row.

$$a_{1,*} = [p_1, p_2, p_3 \ldots p_N]$$
$$Z_1 = \sum_{j=1}^{N} a_{1,j}$$

Step 2: Recursively compute the other elements.

$$
\begin{aligned}
&\text{For } i = 2 \text{ to } N \\
&\qquad a_{i,N} = a_{1,N}(Z_{i-1} - a_{i-1,N}) \\
&\qquad \text{For } j = N - 1 \text{ to } i \text{ Step } -1 \\
&\qquad\qquad a_{i,j} = a_{1,j} \cdot \left( \frac{a_{i,j+1}}{a_{1,j+1}} - a_{i-1,j} \right) \\
&\qquad \text{Next } j \\
&\qquad Z_i = \sum_{j=i}^{N} a_{i,j} \\
&\text{Next } i
\end{aligned}
$$

The matrix for $N = 6$ is given below.

$$\begin{pmatrix} p_1 & p_2 & p_3 & p_4 & p_5 & p_6 \\ 0 & p_2\left(\frac{a_{2,3}}{p_3} - p_2\right) & p_3\left(\frac{a_{2,4}}{p_4} - p_3\right) & p_4\left(\frac{a_{2,5}}{p_5} - p_4\right) & p_5\left(\frac{a_{2,6}}{p_6} - p_5\right) & p_6(Z_1 - p_6) \\ 0 & 0 & p_3\left(\frac{a_{3,4}}{p_4} - a_{2,3}\right) & p_4\left(\frac{a_{3,5}}{p_5} - a_{2,4}\right) & p_5\left(\frac{a_{3,6}}{p_6} - a_{2,5}\right) & p_6(Z_2 - a_{2,N}) \\ 0 & 0 & 0 & p_4\left(\frac{d_{4,5}}{p_5} - a_{3,4}\right) & p_5\left(\frac{d_{4,6}}{p_6} - a_{3,5}\right) & p_6(Z_3 - a_{3,N}) \\ 0 & 0 & 0 & 0 & p_5\left(\frac{d_{5,6}}{p_6} - a_{4,5}\right) & p_6(Z_4 - a_{4,N}) \\ 0 & 0 & 0 & 0 & 0 & p_6(Z_5 - a_{5,N}) \end{pmatrix} \cdot \begin{pmatrix} 1 \\ 1 \\ 1 \\ 1 \\ 1 \\ 1 \end{pmatrix} = \begin{pmatrix} Z_1 \\ Z_2 \\ Z_3 \\ Z_4 \\ Z_5 \\ Z_6 \end{pmatrix}$$

### Example 3.

The *exhaustive method* relies on the explicit calculation of all combinatorial possibilities followed by their summation to derive the joint probability $P_s(n)$ values. This explicit enumeration may be approached in several ways. One example would be by pre-indexing all possible combinations of individual probabilities ($p_{f,s}$) and reciprocal probabilities ($1-p_{f,s}$) beforehand. Another example would be using a self-recursive function which takes the current depth position, the total probability up to that point and the probability level to calculate a new probability using the previous total probability and the current probability value, and which then calls itself until it reaches the total depth. Both approaches do not differ in time complexity. A confidence interval is then created using the previously calculated Z-values and the probabilities, using a middle-out approach. As starting point, the frequency most likely to have the highest probability in the confidence interval is computed. Next, the method calculates probabilities towards each end of the confidence interval until the set confidence interval limit is reached.

The *semi-exhaustive method* approach relies on expressing the joint probability $P_s(n)$ values in terms of Z-values and binomial coefficients C(n,k). The Z-values are then computed not by using the Z data structure, but by explicitly enumerating all combinatorial possibilities of individual probabilities ($p_{f,s}$) contributing to each joint probability $P_s(n)$ value. A confidence interval is then created in the usual manner.

The *non-exhaustive method* also relies on expressing the joint probability $P_s(n)$ values in terms of Z-values and binomial coefficients C(n,k). However, the Z-values are not calculated by explicit enumeration but by self-recursion. For every integer from 1 to the number of probabilities to be computed, a-values are first calculated by using a-values from previous recursion and the probabilities supplied to the method. The Z-values are then computed using a-values, probabilities and previous Z-values. A confidence interval is then created in the usual manner. A flowchart for the non-exhaustive method is presented in Fig 2a.

In Fig 4, the actual computation time in seconds required for calculation of a combinatorial probability distribution by conventional exhaustive enumeration (dashed line), semi-exhaustive enumeration (dotted line) and non-exhaustive binomial tree-based computation using the method of the invention (solid line) are shown as functions of sequence read depth at the base position. Also shown on the same graph are fitted regression curves and their corresponding R-values. Specifically, exhaustive enumeration (approx. $y = 0.00002e^{0.8223x}$) and semi-exhaustive enumeration (approx. $y = 0.00002e^{0.712x}$) both scale exponentially with sequence read depth as compared to the method of invention which computes in polynomial time (approx. $y = 0.00002x^2 - 0.0034x + 0.863$).

### Example 4.

501 exons from 28 genes frequently mutated in cancer *(AKT1, AKT2, AKT3, APC, ATM, BRAF, CCND1, CCNE1, CDKN2A, CTNNB1, EGFR, FBXW7, GNAS, HER2, HER3, HER4, IDH1, KRAS, MET, MRE11A, NF1, PIK3CA, PTEN, SMAD2, SMAD3, SMAD4, STK11, TP53)* were enriched and sequenced in ten breast cancer samples. In parallel, the sequences of protein-coding exons of the target genes were collected in tumor DNA by PCR-coupled Sanger sequencing and Selector enrichment followed by SOLID4 sequencing, respectively. Sequence alignment to the human reference genome (hg19) was performed using Mosaik Aligner version 1.1.0018.

Reference allele ratio confidence intervals were computed at all positions where at least 1% of the observed alleles were different from the most frequently observed allele (Fig 5). Probability density functions for the number of observed bases identical to the reference base at four different positions with 61 (A), 105 (B), 342 (C), and 1496 (D) aligned reads, respectively. The observed allele ratio, derived simply by dividing the number of reference alleles with the total number of observations without considering the alignment quality score, is indicated by a dashed line. In the first example of read depth 61 the null hypothesis that the true allele ratio is 0 cannot be rejected as the lower limit of the confidence interval is 0 (Fig 5A). With increasing sequence depths, the confidence intervals decrease (Fig 5B-D). Note that the calculations in Fig 5A-D are beyond the practical range if conventional combinatorial approaches are employed. When accounting for alignment quality, the observed allele ratios will occasionally fall outside the confidence interval (Fig 5B-C).

The invention is not limited to the embodiments and examples shown, but may be varied freely within the framework of the following claims.

**Claims**

1. A method for determining the presence of a mutation in a DNA/RNA-sample comprising a plurality of DNA/RNA-fragments, the method comprising:

a) storing in a computer memory for each (f) of a number (N) of measured DNA/RNA-fragments in the sample information on:

an individual probability estimation ($p_{f,s}$) that a measured nucleotide base in a position (s) is correctly identified, and on
a match ($m_{f,s}$) between the measured nucleotide base in said position of the measured DNA/RNA-fragment and a reference nucleotide base ($r_s$) in said position (s) of a reference DNA/RNA,

b) estimating with the processor a joint probability $P_s(n)$ of measuring a specific number (n) of matching nucleotide bases at said position (s) by summing the combinations of individual probabilities ($p_{f,s}$) and reciprocal probabilities ($1-p_{f,s}$) that gives that specific number (n) of matches, and
c) determining if a mutation is present by controlling the processor to produce the total number of measured matches ($M=\Sigma_f m_{f,s}$; for s = constant) at said position, and the joint probability $P_s(n)$ of measuring that number (M) of matches at said position.

2. A method according to claim 1, **characterized in that** the method comprises:

- controlling the processor to repeat step b) to estimate at least one additional joint probability $P_s(n)$ of measuring another, specific number (n) of matching nucleotide bases, and
- controlling the processor to sum one or more joint probabilities $P_s(n)$ with different specific numbers (n) of matching nucleotide bases about the measured number of matches (M) to estimate a confidence that a mutation is present.

3. A method according to claim 1 or 2, **characterized in that** in step b) the method comprises:

- allocating a data structure Z in the digital memory having a plurality of data entries ($Z_x$), and
- storing in each data entry ($Z_x$) the combinations of products of individual probability estimations ($p_{f,s}$) comprising the same number (x) of product terms.

4. A method according to claim 3, **characterized in that** in step b) the method comprises:

- storing a combination distribution weight function ($B_{x,n}$) in the computer memory, and
- controlling the processor to estimate the joint probability $P_s(n)$ as a weighted sum of values of the data entries ($Z_x$) of Z multiplied with the combination distribution weight function ($B_{x,n}$).

5. A method according to claim 4, **characterized in that** the method comprises:

- storing a combination distribution weight function ($B_{x,n}$) comprising a series of binomial coefficients with alternating signs in the digital memory.

6. A method according to one of the claims 3, 4 or 5, **characterized in that** the method comprises:

- allocating a data structure A in the digital memory having a plurality of data entries ($a_{x,n}$),
- storing in the data entries ($a_{x,n}$) of the data structure A the value of one combination of terms of the individual probability estimates ($p_{f,s}$), and
- calculating the data entries ($Z_x$) of Z as a sum of values of data entries ($a_{x,n}$) of the data structure A.

7. A method according to claim 6, **characterized in that** the method comprises:

- assigning values to some of the data entries ($a_{x,n}$) of the data structure A by initializing a first row of data entries ($a_{1,n}$) of the data structure A by assigning to each data entry ($a_{1,n}$) the value of one individual probability estimate ($p_{f,s}$).

**8.** A method according to claim 6 or 7, **characterized in that** the method comprises:

- assigning values to some of the data entries ($a_{x,n}$) of the data structure A, and
- controlling the processor to calculate and assign values to the remaining data entries ($a_{x,n}$) of the data structure A by using values of previously initialized data entries ($a_{x,n}$) of the data structure A.

**9.** A method according to claim 8, **characterized in that** the method comprises:

- controlling the processor to calculate and assign values to the remaining data entries ($a_{x,n}$) of the data structure A recursively.

**10.** A method according to claim 1, **characterized in that** the individual probability estimations ($p_{f,s}$) that a measured nucleotide base is correct is determined based on an alignment quality for each DNA/RNA fragment and on a measurement error estimation for each individual nucleotide base position of the DNA/RNA-fragment.

**11.** A method according to any of the claims 1-10, **characterized in that** the method comprises performing the steps a) to c) for a second DNA/RNA-sample comprising DNA/RNA-fragments containing sequences with substantially the same nucleotide base positions as the first sample, and

d) determining if a mutation is present by comparing the results obtained in c) for the first and second samples.

**12.** A method according to claim 11, **characterized in that** the first and second DNA/RNA-samples originates from healthy and tumor tissue from the same individual, respectively, and that the method comprises determining a cancerous state if the nucleotide bases in at least one corresponding position (s) differs between the first and second DNA/RNA-samples within a desired level of confidence.

**13.** A computer program comprising a sequence of computer program instructions adapted to control a computer comprising a processor and at least one computer memory associated with the processor to determine a presence of a mutation in a DNA/RNA-sample comprising a plurality of DNA/RNA-fragments, the computer program controlling the computer to perform, for one or more position(s) of the nucleotide sequences of said DNA/RNA-fragments in the sample, the steps:

a) storing in the computer memory for each (f) of a number (N) of measured DNA/RNA-fragments in the sample information on:

a probability estimation ($p_{f,s}$) that a measured nucleotide base in a position (s) is correctly identified, and on a match ($m_{f,s}$) between the measured nucleotide base in said position of the measured DNA/RNA-fragment and a reference nucleotide base ($r_s$) in said position (s) of a reference DNA/RNA,

b) estimating with the processor a joint probability $P_s(n)$ of measuring a specific number (n) of matching nucleotide bases at said position (s) by summing the combinations of individual probabilities ($p_{f,s}$) and reciprocal probabilities ($1-p_{f,s}$) that gives that specific number (n) of matches, and

c) determining if a mutation is present by controlling the processor to produce the total number of measured matches ($M=\Sigma m_{f,s}$; for s = constant) at said position, and the joint probability $P_s(n)$ of measuring that number (M) of matches at said position.

**14.** A computer program according to claim 13, **characterized in that** the computer program is adapted to control the computer to perform the steps:

- controlling the processor to repeat step b) to estimate at least one additional joint probability $P_s(n)$ of measuring another, specific number (n) of matching nucleotide bases, and
- controlling the processor to sum one or more joint probabilities $P_s(n)$ with different specific numbers (n) of matching nucleotide bases about the measured number of matches (M) to estimate a confidence that a mutation is present.

**15.** A computer program according to claim 13 or 14, **characterized in that** the computer program is adapted to control the computer to perform the steps:

- allocating a data structure Z in the digital memory having a plurality of data entries ($Z_x$), and
- storing in each data entry ($Z_x$) the combinations of products of individual probability estimations ($p_{f,s}$) comprising the same number (x) of product terms.

16. A computer program according to claim 15, **characterized in that** the computer program is adapted to control the computer to perform the steps:

- storing a combination distribution weight function ($B_{x,n}$) in the computer memory, and
- controlling the processor to estimate the joint probability $P_s(n)$ as a weighted sum of values of the data entries ($Z_x$) of Z multiplied with the combination distribution weight function ($B_{x,n}$).

17. A computer program according to claim 15 or 16, **characterized in that** the computer program is adapted to control the computer to perform the steps:

- allocating a data structure A in the digital memory having a plurality of data entries ($a_{x,n}$),
- storing in the data entries ($a_{x,n}$) of the data structure A the value of one combination of terms of the individual probability estimates ($p_{f,s}$), and
- calculating the data entries ($Z_x$) of Z as a sum of values of data entries ($a_{x,n}$) of the data structure A.

18. A computer program according to claim 17, **characterized in that** the computer program is adapted to control the computer to perform the step:

- assigning values to some of the data entries ($a_{x,n}$) of the data structure A by initializing a first row of data entries ($a_{1,n}$) of the data structure A by assigning to each data entry ($a_{1,n}$) the value of one individual probability estimate ($p_{f,s}$).

19. An electronic device comprising analog and/or digital electronic circuitry for determining the presence of a mutation in a DNA/RNA-sample comprising a plurality of DNA/RNA-fragments, the device comprising:

- a first storage module adapted to store, for each (f) of a number (N) of measured DNA/RNA-fragments in the sample, information representing an individual probability estimation ($p_{f,s}$) that a measured nucleotide base in a position (j) is correctly identified, and information representing a match ($m_{f,s}$) between the measured nucleotide base in said position of the measured DNA/RNA-fragment and a reference nucleotide base ($r_s$) in said position (s) of a reference DNA/RNA,
- an estimation module adapted to estimate a joint probability $P_s(n)$ of measuring a specific number (n) of matching nucleotide bases at said position (s) by summing the combinations of individual probabilities ($p_{f,s}$) and reciprocal probabilities ($1-p_{f,s}$) that gives that specific number (n) of matches, and
- a determination module adapted to determine if a mutation is present by producing the total number of measured matches ($M=\Sigma_f m_{f,s}$; for s = constant) at said position with the joint probability $P_s(n)$ of measuring that number (M) of matches at said position.

**Fig 1**

Start

$i=j=1, b=0, Z_0=1$
$a=array.shape(N-1,N-1)$
$a_{0,0}...a_{0,N-1} = p_1...p_N$

$j=j+1$
$J=1$

$a_{i,j}=a_{0,n} \sum_{n=0}^{j-1} a_{i-1,n}$

$i=i+1$

$i<j$ — Yes

No

$j<N-1$

Yes

Calculate for $i=1$ to $N$
$Z_i=\sum_{j=i}^{N} a_{i,j}$

End

**Forward**

$\square = \boxed{\square} \times \boxed{\Sigma}$

| $p_1$ | $p_2$ | $p_3$ | $\boxed{p_4}$ | $p_5$ | $p_6$ |
|---|---|---|---|---|---|
| 0 | $a_{2,2}$ | $a_{2,3}$ | $a_{2,4}$ | $a_{2,5}$ | $a_{2,6}$ |
| 0 | 0 | $a_{3,3}$ | $a_{3,4}$ | $a_{3,5}$ | $a_{3,6}$ |
| 0 | 0 | 0 | $a_{4,4}$ | $a_{4,5}$ | $a_{4,6}$ |
| 0 | 0 | 0 | 0 | $a_{5,5}$ | $a_{5,6}$ |
| 0 | 0 | 0 | 0 | 0 | $a_{6,6}$ |

**Fig 2a**

Start

$i=j=1, b=0, Z_0=1$
$a=array.shape(N,N)$
$a_{0,0}...a_{0,N-1} = p_1...p_N$

$j=j+1$
$j=i$
$b = 0$

$b = b + a_{i-1,j-1}$
$a_{i,j} = ba_{0,j}$

$j=j+1$

$j<N-1$   Yes

No

Yes   $i<N-1$

No

Calculate for i = 1 to N
$Z_i = \sum_{j=i}^{N} a_{i,j}$

End

$\square = \left(\boxed{\Sigma} - \left[\vdots\Sigma\vdots\right]\right) \times \square$

**Reverse**

| $p_1$ | $p_2$ | $p_3$ | $\boxed{p_4}$ | $p_5$ | $p_6$ |
|-------|-------|-------|-------|-------|-------|
| 0 | $a_{2,2}$ | $a_{2,3}$ ← | $a_{2,4}$ | $a_{2,5}$ | $a_{2,6}$ |
| 0 | 0 | $a_{3,3}$ | $a_{3,4}$ | $a_{3,5}$ | $a_{3,6}$ |
| 0 | 0 | 0 | $a_{4,4}$ | $a_{4,5}$ | $a_{4,6}$ |
| 0 | 0 | 0 | 0 | $a_{5,5}$ | $a_{5,6}$ |
| 0 | 0 | 0 | 0 | 0 | $a_{6,6}$ |

**Fig 2b**

Start

$i=j=1, b=0, Z_0=1$
$a=array.shape(N,N)$
$a_{0,0}...a_{0,N-1} = p_1...p_N$

Calculate in parallel for k = 1 to j
$Z_k = Z_k + a_{k-1,j-1}$
$a_{k,j} = Z_k a_{0,j}$

$j=j+1$

$j<N-1$

Yes

No

Calculate in parallel for k = 1 to N
$Z_k = Z_k + a_{k-1,j-1}$

End

**Parallel**

| $p_1$ | $p_2$ | $p_3$ | $p_4$ | $p_5$ | $p_6$ |
|---|---|---|---|---|---|
| 0 | $a_{2,2}$ | $a_{2,3}$ | $a_{2,4}$ | $a_{2,5}$ | $a_{2,6}$ |
| 0 | 0 | $a_{3,3}$ | $a_{3,4}$ | $a_{3,5}$ | $a_{3,6}$ |
| 0 | 0 | 0 | $a_{4,4}$ | $a_{4,5}$ | $a_{4,6}$ |
| 0 | 0 | 0 | 0 | $a_{5,5}$ | $a_{5,6}$ |
| 0 | 0 | 0 | 0 | 0 | $a_{6,6}$ |

**Fig 2c**

$$\text{Start}$$

$$i=0$$

$$i=i+1 \rightarrow P(i)_j = \sum_{n=0}^{N} \left(\frac{n}{n-i}\right) Z_n (-1)^{n-i}$$

$$i < N$$

Yes

No

$$\text{End}$$

$$
\begin{pmatrix}
\binom{0}{0} & -\binom{1}{1} & \binom{2}{2} & -\binom{3}{3} & \binom{4}{4} & -\binom{5}{5} & \binom{6}{6} \\
0 & \binom{1}{0} & -\binom{2}{1} & \binom{3}{2} & -\binom{4}{3} & \binom{5}{4} & -\binom{6}{5} \\
0 & 0 & \binom{2}{0} & -\binom{3}{1} & \binom{4}{2} & -\binom{5}{3} & \binom{6}{4} \\
0 & 0 & 0 & \binom{3}{0} & -\binom{4}{1} & \binom{5}{2} & -\binom{6}{3} \\
0 & 0 & 0 & 0 & \binom{4}{0} & -\binom{5}{1} & \binom{6}{3} \\
0 & 0 & 0 & 0 & 0 & \binom{5}{0} & -\binom{6}{1} \\
0 & 0 & 0 & 0 & 0 & 0 & \binom{6}{0}
\end{pmatrix}
\cdot
\begin{pmatrix}
Z_0 \\ Z_1 \\ Z_2 \\ Z_3 \\ Z_4 \\ Z_5 \\ Z_6
\end{pmatrix}
=
\begin{pmatrix}
P(0) \\ P(1) \\ P(2) \\ P(3) \\ P(4) \\ P(5) \\ P(6)
\end{pmatrix}
$$

**Fig 3**

$$y = 2E\text{-}05x^2 - 0.0034x + 0.863$$
$$R^2 = 0.9997$$

$$y = 3E\text{-}05e^{0.712x}$$
$$R^2 = 0.9983$$

$$y = 2E\text{-}05e^{0.8223x}$$
$$R^2 = 0.9922$$

**Fig 4**

**Fig 5**

Fig 6

Generate confidence intervals for genome database reference allele ratio in tumor (T) and matched normal (N) sample. Can 1 be excluded (+) or not from (-) from the confidence interval?

| N+, T+ | N+, T- | N-, T+ | N-, T- |

Difference    Difference    No Difference

Create confidence intervals of allele ratio of the most frequent allele in N. Can 1 be excluded (+) or not (-) from the confidence interval?

| N+, T+ | N+, T- | N-, T+ | N-, T- |

Difference    Difference    No Difference

Compare the second most frequen alleles in T and D. Do they differ?

| Yes | No |

Difference    No Difference

**Fig 7**

Fig 8

**EUROPEAN SEARCH REPORT**

Application Number

EP 12 17 2544

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | A. NIIDA ET AL: "Statistical model-based testing to evaluate the recurrence of genomic aberrations", BIOINFORMATICS, vol. 28, no. 12, 15 June 2012 (2012-06-15), pages i115-i120, XP55042795, ISSN: 1367-4803, DOI: 10.1093/bioinformatics/bts203 * the whole document * | 1-19 | INV. G06F19/22 G06F19/24 |
| A | Yili Hong: "On computing the distribution function for the sum of independent and non-identical random indicators", Technical Report 11-3, 5 April 2011 (2011-04-05), pages 1-17, XP55042798, Department of Statistics, Virginia tech, Blacksburg, USA Retrieved from the Internet: URL:http://www.web-e.stat.vt.edu/dept/web-e/tech_reports/Technical_Report_11_2.pdf [retrieved on 2012-10-31] * page 1 - page 7 * | 1-19 | |
| A | CHEN: "Statistical Applications of the Poisson-Binomial and conditional Bernoulli distributions", STATISTICA SINICA, vol. 7, 1 January 1997 (1997-01-01), page 875, XP55042807, ISSN: 1017-0405 * page 875 - page 880 * | 1-19 | |

TECHNICAL FIELDS SEARCHED (IPC)

G06F

-/--

The present search report has been drawn up for all claims

| Place of search | Date of completion of the search | Examiner |
|---|---|---|
| Munich | 7 November 2012 | Kürten, Ivayla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 12 17 2544

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | I. CHEPELEV ET AL: "Detection of single nucleotide variations in expressed exons of the human genome using RNA-Seq", NUCLEIC ACIDS RESEARCH, vol. 37, no. 16, 1 September 2009 (2009-09-01), pages e106-e106, XP55042801, ISSN: 0305-1048, DOI: 10.1093/nar/gkp507 * the whole document * | 1-19 | |
| A | E. R. MARTIN ET AL: "SeqEM: an adaptive genotype-calling approach for next-generation sequencing studies", BIOINFORMATICS, vol. 26, no. 22, 15 November 2010 (2010-11-15), pages 2803-2810, XP55042804, ISSN: 1367-4803, DOI: 10.1093/bioinformatics/btq526 * page 2804, paragraph 2 - page 2805 * | 1-19 | |
| L | A. WILM ET AL: "LoFreq: a sequence-quality aware, ultra-sensitive variant caller for uncovering cell-population heterogeneity from high-throughput sequencing datasets", NUCLEIC ACIDS RESEARCH, 1 January 2012 (2012-01-01), XP55042810, ISSN: 0305-1048, DOI: 10.1093/nar/gks918 * the whole document * | 1-19 | TECHNICAL FIELDS SEARCHED (IPC) |

The present search report has been drawn up for all claims

| Place of search | Date of completion of the search | Examiner |
|---|---|---|
| Munich | 7 November 2012 | Kürten, Ivayla |

EPO FORM 1503 03.82 (P04C01)